# EUROPEAN PATENT SPECIFICATION

(11) **EP 3 978 151 B1**
(45) Date of publication and mention of the grant of the patent: **30.07.2025**
(21) Application number: 21198334.1
(22) Date of filing: 22.09.2021
(51) Int. Cl.: B08B 7/00, A61L 2/10, G01N 21/64, G01N 31/22

(54) **SYSTEMS AND METHODS OF UV CLEANING**
SYSTEME UND VERFAHREN ZUR UV-REINIGUNG
SYSTÈMES ET PROCÉDÉS DE NETTOYAGE UV

(30) Priority: 30.09.2020 US 202063085440 P; 31.08.2021 US 202117462911
(43) Date of publication of application: 06.04.2022
(73) Proprietor: The Boeing Company, Arlington, VA 22202 (US)
(72) Inventor: MCINTOSH, Darren C., CHICAGO, 60606-2016 (US)
(74) Representative: Plasseraud IP

(56) References cited:
- US-A1- 2001 048 891
- US-A1- 2015 343 102
- US-A1- 2018 369 440
- US-A1- 2019 351 086

## Description

### BACKGROUND

Current surface cleaning methods may not provide reliable indications that the cleaning process has been performed. Accordingly, neither the users of these surfaces nor those responsible for performing the cleaning processes may reliably confirm that the surfaces have been sufficiently cleaned. Accordingly, there remains a need for systems and methods to reliably clean and confirm that cleaning procedures have been executed.

US 2018/369440 A1, in accordance with its abstract, states a cleanliness indication system and a method that indicate that a surface of a component is clean. The cleanliness indication system and method include a photoluminescent indicator that is configured to be on the surface of the component. The photoluminescent indicator is configured to be exposed to ultraviolet (UV) light during a UV light sanitizing cycle. The photoluminescent indicator emits light due to exposure to the UV light. The photoluminescent indicator indicates that the surface of the component is clean by emitting the light due to exposure to the UV light.

US 2019/351086 A1, in accordance with its abstract, states a method and an apparatus for indicating minimum exposure of a surface to UVC light emitted by a source during a decontamination process. The apparatus includes a photochromic material to be applied or adjacent to the surface. The photochromic material is to exhibit a visible response to receiving the minimum exposure to the UVC light, and exhibit the visible response to a lesser extent after the photochromic material ceases to be exposed to the UVC light emitted by the source. A protective layer of material that is substantially transparent to the UVC light emitted by the source is positioned over the photochromic material to be disposed between the source and the photochromic material during the decontamination process.

US 2015/343102 A1, in accordance with its abstract, states methods for detecting electromagnetic energy (e.g., ultraviolet radiation) and articles suitable for use in such methods. The methods and articles are for detecting sterilization or disinfection resulting from electromagnetic energy such as ultraviolet C. Detection of the sterilization or disinfection allows discontinuation of the exposure when a desired level of sterilization or disinfection has been obtained. Feedback mechanisms for controlling exposure to the energy may also be used.

US 2001/048891 A1, in accordance with its abstract, states a photochromic material which undergoes a change in appearance when exposed to ultraviolet (UV) radiation in the UVC band to provide a visual indication of the degree of sterilization achieved by a UV emitting device. The photochromic material is placed either on the UV source or in or adjacent the sample being sterilized. The photochromic material is formulated to undergo a certain degree of change in appearance when it has been exposed to a level of UV which corresponds to effective sterilization of the sample being sterilized. An optional reference card bears a color or opacity reference for comparison with the exposed photochromic material.

### SUMMARY

The present disclosure provides an assembly defined in claim 1, and a system defined in claim 10.

In one aspect, in combination with any example above or below, the system or assembly may include that the component is formed from a material selected from the group consisting of: a polymer, a polycarbonate, a metal, a composite, a nano-reinforced material, an organic material, a textile fabric, and combinations thereof.

In one aspect, in combination with any example above or below, the system or assembly may include that the photochromatic indicator is in a form selected from the group consisting of: a sticker, a thread, a textile fabric, an organic material, a polycarbonate element, a polymer element, a metal element, a coating, and combinations thereof.

In one aspect, in combination with any example above or below, the system or assembly may include that photochromatic indicator is included in a tray cover, a headrest cover, a seat cover, an armrest cover, an outlet cover, or a headset cover.

In one aspect, in combination with any example above or below, the system or assembly may include that the photochromatic indicator includes a reversible photochromatic pigment.

In one aspect, in combination with any example above or below, the system or assembly may include that the photochromatic indicator includes an irreversible photochromatic pigment.

In one aspect, in combination with any example above or below, the system may include that the UV light source includes a wireless device, a wired device, a device removably coupled to a wall of an aircraft passenger cabin, a device removably coupled to a ceiling of an aircraft passenger cabin, a device removably coupled to a seat of an aircraft passenger cabin, or a device removably coupled to a floor of an aircraft passenger cabin.

In one aspect, in combination with any example above or below, the system or assembly may include that the photochromatic indicator is not visible to the naked eye when in the first state.

In one aspect, in combination with any example above or below, the system may include that the UV light source emits a wavelength of from about 222 to about 254 nanometers (nm).

In one aspect, in combination with any example above or below, the system or assembly may include that the photochromatic indicator is included in a cover comprising at least one aperture, the cover being configured to couple to the component through one or more of an adhesive, a magnetic means, a mechanical means, a hook-and-loop means, or combinations thereof.

In one aspect, in combination with any example above or below, the system or assembly may include that the component comprises a gaming system The present disclosure provides a method as defined in claim 13.

In one aspect, in combination with any example method above or below, the method may include that the photochromatic indicator is included in a single-use component removably coupled to the component through an adhesive, a magnetic means, a hook-and-loop means, a mechanical means, or combinations thereof.

In one aspect, in combination with any example method above or below, the method may include that the photochromatic indicator includes two or more photochromatic pigments, each of the two or more photochromatic pigments having an at least one of a different concentration or a different composition.

The present disclosure provides a method as defined in claim 14.

In one aspect, in combination with any example method above or below, the method may include that, prior to applying the UV light source, removably coupling the first photochromatic indicator to the component, the first photochromatic indicator including irreversible photochromatic pigment.

In one aspect, in combination with any example method above or below, the method may include (b) re-applying the UV light source to the first photochromatic indicator subsequent to the first photochromatic indicator changing from the second state to the first state. The first photochromatic indicator changes from the first state back to the second state in response to (b), indicating that the component has been cleaned.

In one aspect, in combination with any example method above or below, the method may include performing (a) and (b) for a plurality of iterations, and the first photochromatic indicator includes at least one reversible pigment.

In one aspect, in combination with any example method above or below, the method may include, subsequent to performing (a) and (b) for a plurality of iterations: uncoupling the first photochromatic indicator from the component; disposing of the first photochromatic indicator; and associating a second photochromatic indicator with the component, the second photochromatic indicator being in the first state.

In one aspect, in combination with any example method above or below, the method may include that, the first photochromatic indicator comprises a brand identifier.

In one aspect, in combination with any example assembly above or below, the photochromatic indicator may be configured as a disposable, single-use element.

The present disclosure may further provide, as a non-claimed example, a photochromatic indicator, the photochromatic indicator including: a photochromatic pigment, the photochromatic pigment being in a first state under ambient light and configured to be in a second state under ambient light after being exposed to a UV light source, the first state being different than the second state, the photochromic pigment being configured to change from the first state to the second state in response to the UV light source.

### BRIEF DESCRIPTION OF THE DRAWINGS

So that the manner in which the above recited features can be understood in detail, a more particular description, briefly summarized above, may be had by reference to example aspects, some of which are illustrated in the appended drawings.
FIG. 1 depicts a system configured to clean one or more components according to aspects of the present disclosure.
FIG. 2 is an illustration of an example photochromatic indicator according to aspects of the present disclosure.
FIG. 3 is a flow chart of a method of cleaning using photochromatic indicators according to aspects of the present disclosure.
FIG. 4 is a flow chart of a method of cleaning using photochromatic indicators according to aspects of the present disclosure.
FIGS. 5A-5C depict a photochromatic indicator at various operations of a cleaning method according to aspects of the present disclosure.
FIGS. 6A-6F depict another photochromatic indicator at various operations of a cleaning method according to aspects of the present disclosure.

### DETAILED DESCRIPTION

The present disclosure relates to the cleaning, e.g., the disinfection and sanitization of one or more surfaces of a component using ultraviolet (UV) light. The cleaning discussed herein can, in some examples, include sterilization. As used herein, "sterilization" is a process that makes one or more surfaces of a component free from bacteria, viruses, or other living microorganisms. The surfaces discussed herein may be "high-touch" surfaces contacted by multiple parties, such as seats, trays, counters, touch screens, keyboards, or gaming consoles, in contrast with "low-touch" surfaces such as ceilings or other harder-to-reach surfaces. In some examples, such "high-touch" surfaces may be present on an aerospace vehicle such as a commercial airplane. In some examples, UV light may be used alone, and in other examples, UV light may be used in combination with one or more cleaning solvents to remove contaminants such as dirt and oils as well as biologic elements such as viruses and bacteria such that the cleaned surfaces are not likely to transmit biologic contaminants among and between users of the high-touch surfaces.

In currently employed cleaning processes, a photo-luminescent element or elements can be used to indicate that rooms and surfaces, such as lavatories on aircrafts, have been cleaned. However, currently employed photo-luminescent processes may be commonly confirmed in a darkened passenger cabin, or other areas capable of having all light sources deactivated or otherwise covered, where photo-luminescent strips emit light from absorbed photons. In addition, some currently employed systems use photo-luminescent indicators that can be charged from a multitude of light sources such as ambient light sources in addition to cleaning sources such as UV lights. As such, the photo-luminescent indicators might glow from being charged by these light sources, thereby undesirably providing a false reading of ultraviolet (UV) exposure and resultant clean status.

The high-touch surfaces discussed herein can include components of aircrafts, such as those in the passenger cabin, crew areas, or cockpit, as well as gaming systems. As discussed herein, "gaming systems" are systems configured for gambling in casinos or for non-gambling environments such as all-ages arcades or home systems, including e-sports systems. It is further contemplated that other high-touch surfaces in high-traffic areas such as touch-screen and/or keypads, card readers, or other surfaces or combinations of surfaces in retail stores, shopping malls, schools, offices, rental cars, ride shares, housing shares, financial institution, automated teller machines (in financial institutions or other locations), hospital, hotels/motels/resorts, or other locations could employ the systems and methods discussed herein to clean both high-touch surfaces as well as other areas. These high-touch surfaces may further include door handles, desktops, countertops, key boards, computer mice, touch screens/GUIs on kiosks or mobile devices, other areas of mobile devices and/or mobile device cases, clothes hangers, seating, cutlery, dishware, glassware, or other items including surfaces that may be touched by multiple parties during the course of use.

Using the systems and methods discussed herein, photochromatic indicators are used to indicate, to a party responsible for cleaning a component as well as to a party who may use the component, when the component including one or more high-touch surfaces have been cleaned. The systems and methods discussed herein thus instill confidence and, in some examples, confirmation and auditability that cleaning has been performed. The cleaning can be defined by a predetermined schedule set by a party who owns or rents the component(s), or by a local, state, or federal government regulation.

As used herein, a "photochromatic indicator" is a single-use, multi-use, semi-permanent, or permanent element including one or more photochromatic pigments. Photochromatic pigments are configured to change from a first state to a second state for a predetermined period of time in response to absorbing UV light. For example, photochromatic pigments may change from clear to color upon the absorption of certain UV light. The photochromatic pigments can be in the forms of liquid, solid (e.g., powder), colloids (e.g., slurry), or other forms or combinations of forms. Depending upon the example, the photochromatic pigments can be used in indicators as liquid or gel reservoirs, paints or other coatings, incorporated into stickers, added to polymers, polycarbonates, metals, or textile elements. As used herein, the terms "paint" and "coating" may be used interchangeably to mean various types of materials (such as photochromatic indicators) applied in one or more layers to one or more surfaces of a component that can be removed by chemical, mechanical, or combination means and replaced.

The first states and the second states of photochromatic indicators discussed herein can be confirmed by and are visible using the naked eye in ambient light, such that the area including the photochromatic indicator may not be darkened in order to confirm cleaning. As used herein, "ambient light" is light present in a room from natural light (e.g., the sun), or existing room lighting, in contrast to lights used to illuminate particular aspects of or areas within room or other environment. As used herein, something that is "visible to the naked eye" can be seen without magnification, including when a person or persons are wearing prescription glasses. In other aspects of the present disclosure, the change from the first state to the second state can be confirmed via an application on a mobile device or via a tactile change, for example, if a party who desires to confirm the change is vision-impaired or desires to create a record of the change using the mobile device. The ability to verify that a surface has been cleaned using the photochromatic indicators discussed herein provides assurance for the party cleaning the surface as well as for the party using the surface.

Depending upon the example, the photochromatic pigment(s) included in the photochromatic indicators can be selected and configured to be reversible or irreversible. As used herein in connection with photochromatic indicators including photochromatic pigment, for example, where polymers, polycarbonate, metallic, or textile elements or coatings on elements have photochromatic pigment included therein, "reversible" is to mean that UV light is applied to the photochromatic indicator to change it from a first state to a second state. In this example, after a predetermined period of time that may range from about 10 minutes to about 24 hours, the photochromatic indicator changes from the second state back to the first state. In other examples, the predetermined period of time can be from about 30 minutes to about 3 hours. In still other examples, the predetermined period of time of the first state can be from about 2 hours to about 6 hours. As used herein, "about" is to mean within +/-5% of a stated target value, maximum, or minimum value.

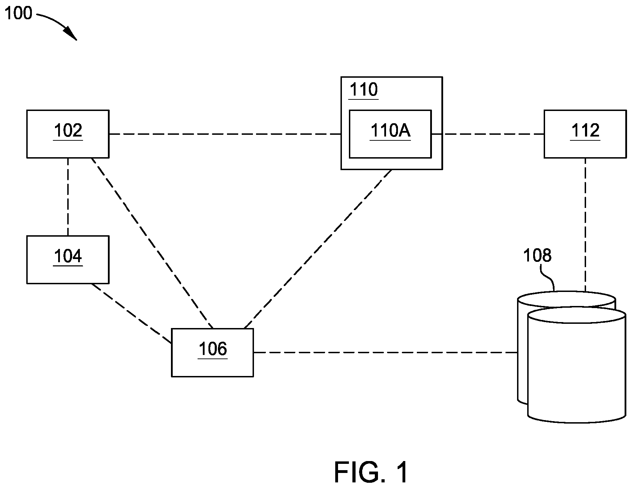

In one example, the first state of a photochromatic indicator can include text and/or a graphic in a first color visible to the naked eye. In another example, which can be combined with other examples herein, the first state of a photochromatic indicator may be invisible to the naked eye. In some examples, the first state can alternatively or additionally include a tactile element such that the photochromatic indicator has one tactile configuration in the first state and a different tactile configuration (e.g., bumps, grooves, or smoothness) in the second state. In some examples, the second state can include a second, different color visible to the naked eye. The second state can include a graphic and/or text indicating that the associated component or surface has been cleaned. In one example, which can be combined with other examples herein, the second state may further include a brand identification, such that the brand identification (logo) is visible in the second state but not in the first state. In some examples, the first state of a photochromatic indicator can be a first color and the second state can be a different color such that the photochromatic indicator is visible in both states. In other examples, the first state of a photochromatic indicator can include a brand in a first color and the second state can include the brand in a different color as well as additional graphics and/or text that were not visible in the first state. The additional graphics and/or text may indicate the component associated with the photochromatic indicator has been cleaned. As discussed herein, a photochromatic indicator can be "associated with" a component by being removably coupled, a permanently coupled, or formed integrally with the component, as discussed below in FIG. 1.

In some examples, photochromatic indicators can be associated with multi-use or semi-permanent components such as an insert in a tray table or an armrest in the passenger cabin or cockpit of an aircraft. These may be referred to as "multi-use" photochromatic indicators since these indicators can be changed from the first state to the second state in response to the absorption of UV light, and then change back to the first state as the UV energy absorbed dissipates, the UV light can then be reapplied to again change the photochromatic indicator from the first state back to the second state. After a predetermined number of re-cleanings, the multi-use photochromatic indicator can be removed, disposed of, refurbished and recycled, or replaced with a new, previously unused photochromatic indicator. Similarly, semi-permanent photochromatic indicators may be changed from the first state to the second state for a plurality of iterations before being eventually replaced during refurbishment of an associated component. For example, in an aircraft, where seating may be replaced or refurbished according to a maintenance schedule, the photochromatic indicator may be included in the fabric or other material comprising the seat, and may be refurbished as a part of the maintenance process. An example of a permanent component where a photochromatic indicator may be used are those that are typically replaced entirely as opposed to being repainted/recoated, re-polished, or otherwise refurbished, due to damage or predetermined maintenance procedures, redesigns, mergers or acquisitions (where branding may change), or other policies or regulations. The photochromatic pigment or elements (e.g., the photochromatic indicator) including the photochromatic pigment(s) should be chosen to be stable under various environmental conditions on an airplane during flight.

Further as used herein in connection with photochromatic pigment or elements including photochromatic pigment, for example, where polymers, polycarbonate, metallic, or textile elements have photochromatic pigment included therein, "irreversible" is to mean that UV light is applied to the photochromatic indicator to change it from a first state to a second state, and does not change back to the first state during the useful life of the photochromatic indicator. That is, the photochromatic indicator including irreversible pigment is removed/refurbished or disposed of prior to the photochromatic indicator changing back to the first state. In some examples, irreversible photochromatic indicators of pigment or including pigment can be included in elements that are single-use, disposable components. These single-use, disposable components may include headrest covers, tray table covers, table covers (e.g., in restaurants or food courts), seat covers, covers for components in lavatories, or other components as appropriate for various applications. The second state may also reflect brands and/or messages indicating the UV-treated status and/or that the photochromatic indicator and/or the component it is associated with is single-use (e.g., a headrest or tray table cover for passenger or pilot seating on an aircraft, bus, or train).

FIG. 1 depicts a system 100 configured to clean one or more components according to examples of the present disclosure. The system 100 can include a UV light source 102 and a power source 104. The UV light source 102 can be configured as a stand-alone, wireless device that includes the power source 104 which may be a rechargeable battery. As discussed in detail below, the UV light source 102 can be configured to emit wavelengths from about 100 nanometers (nm) to about 400 nm. In some examples, the UV light source can be configured to emit wavelengths from about 222 nm to about 254nm. In some examples, the UV light source 102 can have an elongated geometry referred to as a "wand," a circular geometry, polygonal geometry, or can have a combination of two or more geometries. The UV light discussed herein can be UVA, UVB, UVC, or combinations of UV light types. The UV light source 102 can include a plurality of LED lights. In other examples, the UV light source 102 can be configured as a wired device that may or may not be connected to the power source 104 when in use. In still other examples, the UV light source 102 can be configured as a self-driven or manually-driven device or collection of devices that can be coupled to a ceiling of an area to be cleaned and/or a floor of an area to be cleaned. In other examples, the UV light source 102 can be included in a robotically or manually-driven cleaning device that may or may not include the power source 104. In one example, the UV light source 102 is included in a device that can be programmed to move along a predetermined path to clean a plurality of surfaces.

The UV light source 102 can be configured to clean a component 110 having one or more surfaces. As discussed above, the component 110 can be located in an airplane, car, train, bus, office, restaurant, retail location, residence, hotel/resort, hospital, or other location. A photochromatic indicator 110A can be associated with the component 110 to form an assembly. A "removably coupled" photochromatic indicator 110A can be coupled to and removed from the component 110 without causing damage to the component 110. For example, a cover, a sticker, paint/coating, or other forms of photochromatic indicators 110A. A removably coupled photochromatic indicator 110A can also be a semi-permanent photochromatic indicator 110A that can be used in a plurality of cleaning cycles as discussed herein, such that after the semi-permanent photochromatic indicator changes from the first state to the second state and back to the first state a predetermined number of times, it is replaced with a new photochromatic indicator. A "permanently coupled" photochromatic indicator 110A can be formed separately from and then subsequently coupled to the component 110 and used a plurality of times but cannot be removed from the component 110 without causing damage to the component 110. This may be, for example, a thread or threads in the form of a woven textile (e.g., a fabric) that are associated with a seat or headrest subsequent to fabrication of the seat or headrest. An "integral" photochromatic indicator 110A can be molded, cast, or otherwise incorporated into the component 110. The type of component 110 and the type of maintenance performed on the component 110 may enable the integral photochromatic indicator 110A to be removed and replaced or refurbished/recycled. Accordingly, the photochromatic indicator 110A can be in the form of a sticker, a thread, a textile fabric, an organic material (e.g., a wood- or plant-based material such as paper), a polycarbonate element, a polymer element, a metal element, a coating, and combinations thereof

The UV light source 102 can be configured to operate at a wavelength or within a range such that the one or more photochromatic pigments included in the photochromatic indicator 110A change from a first state to a second state in response to absorbing the energy from the UV light source 102. Depending upon the example, the one or more photochromatic pigments included in the photochromatic indicator 110A can be reversible or irreversible as discussed above. In some examples, the photochromatic indicator 110A can be configured to include two or more types (colors) of photochromatic pigment or two or more concentrations of the same type (color) of photochromatic pigments such that the photochromatic indicator 110A has a color gradient formed over time in one or more directions. In one example, the color gradient could be configured to indicate to a cleaner or cleaning system sensor when the photochromatic indicator 110A is changing from the first state to the second state. In another example, which can be combined with other examples herein, the color gradient could be configured to indicate to a cleaner or cleaning system sensor or to a user of the associated component 110 when the photochromatic indicator 110A is changing from the second state back to the first state as an early warning that it may be desirable to re-clean the component 110.

The photochromatic indicator 110A can be formed from one or more materials configured to be used and approved for use in one or more of an aircraft, automobile, hospital, restaurant, retail location, hotel/resort, or other locations as discussed herein. In some locations, the photochromatic indicator 110A will thus be formed from one or more materials configured to resist humidity, fire, wide pH ranges, heat, cold, or other conditions without detaching or uncoupling from an associated component. In some examples, the photochromatic indicator 110A is formed from a material chosen to prevent the release of toxic fumes during a fire.

The component 110 can be formed from one or more of a polymer, a polycarbonate, a metal, a composite, a nano-reinforced material, an organic material (including plant- or wood-based materials such as paper), a textile fabric, and combinations of materials. In various examples, the component 110 can include a tray cover, a headrest cover, a seat cover, an armrest cover, an outlet cover, a headset cover, a keyboard or computer mouse, a table, a seat, a countertop, a touch-screen or other element of a mobile device or kiosk, a tray insert, an armrest insert, a seat insert, or a cover configured to cover one or more surfaces of a gaming system including casino or e-sports gaming systems.

In some examples, the system 100 includes a server computer 106 having a non-transitory memory configured to store a plurality of logic in the form of cleaning programs, cleaning schedules, and cleaning records. This information and other information may be stored on one or more data stores 108 which may be part of a cloud computing system. The UV light source 102 and the power source 104 may be in communication with and/or controlled by the server computer 106. In some examples, a mobile device 112 such as a phone, tablet, personal data assistant, laptop computer, or wearable smart device can include an app configured to determine when the photochromatic indicator 110A is in the first state or the second state.

The system 100 can be used in a variety of cleaning methods with different types of photochromatic indicators associated with varying types of components, including as discussed below.

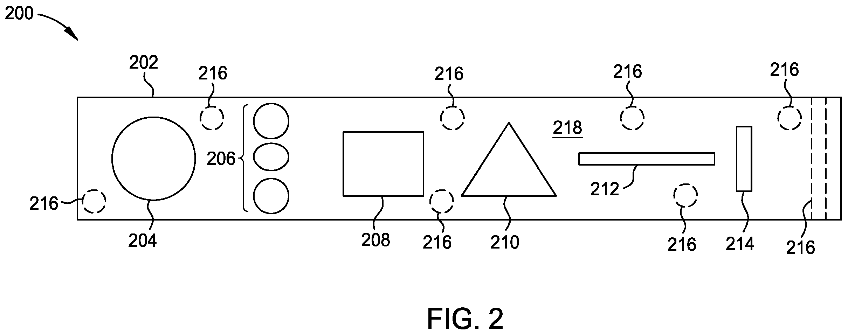

FIG. 2 is an illustration of an example photochromatic indicator 200 according to examples of the present disclosure. As discussed above, photochromatic indicators can take various geometric and material forms, and may be single-use, multi-use, semi-permanent, or permanent. The photochromatic indicator 200 is defined in part by a contiguous edge 202. In some examples, the contiguous edge 202 can be a reinforced material including a polymer, polycarbonate, metal, or other materials or combinations of materials. The example photochromatic indicator 200 includes a plurality of apertures of various geometries, including a circular aperture 204, a polygonal aperture 208, a triangular aperture 210, a horizontal slot aperture 212, and a vertical slot aperture 214. The photochromatic indicator 200 further includes a plurality of apertures 206 of varying geometries that can be configured as a cluster.

In one example, the photochromatic indicator 200 can be formed from organic materials such as wood or paper, polymer, polycarbonate, metal, composite, or combinations of materials. One or more photochromatic indication points 216 can be located in various places on the photochromatic indicator 200, each including one or more photochromatic pigments that change from a first state to a second state in response to application of a UV light source as discussed above. In other examples, the entire surface 218 of the photochromatic indicator 200 includes one or more photochromatic pigments, possibly in gradient form, such that the entire surface 218 changes from a first state to a second state in response to application of the UV light source. The photochromatic indicator 200 can include branding specific to a casino, an e-sports brand, restaurant, or bar including an arcade, a bank, or other institution that employs the device to indicate that the component it is associated with has been cleaned.

The photochromatic indicator 200 can be coupled to a component using an adhesive, a mechanical means, a magnetic means, a hook-and-loop means, a friction or press-fit-based means, or combinations thereof. Each of the apertures 204, 206, 208, and 210 could be configured to allow access to a control or selection feature of the associated component. In another example, one of more of the horizontal slot aperture 212 and the vertical slot aperture 214 could be configured to accept card-present transactions from various payment methods including gaming cards. In another example, one or more of the apertures 204, 206, 208, and 210 or the slot apertures 212 and 214 could be configured to enable RFID or other wireless communications such as receiving information from a payment instrument (virtual or physical), gaming card or app, or other payment instrument.

The photochromatic indicator 200 could be a part of a system such as the system 100, and may be configured to include reversible or irreversible pigment(s) or gradients of pigment, depending upon the example. In some examples, a gradient can include a combination of both irreversible and reversible photochromatic pigments. In some examples, a gradient can include only irreversible or reversible photochromatic pigments. In one example, the photochromatic indicator 200 can be removably coupled to a slot machine or other gambling machine in a casino or other location where gambling is facilitated. In another example, the photochromatic indicator 200 could be coupled to gaming equipment in an arcade outside of a casino, for example, an e-sports location. While the photochromatic indicator 200 is shown here as being a strip configuration, in other examples, it could be configured to wrap around handles, steering wheels, joysticks, or other control systems used for gaming where it is desirable for both the equipment owners and users to be able to confirm that the component associated with the photochromatic indicator 200 has been cleaned.

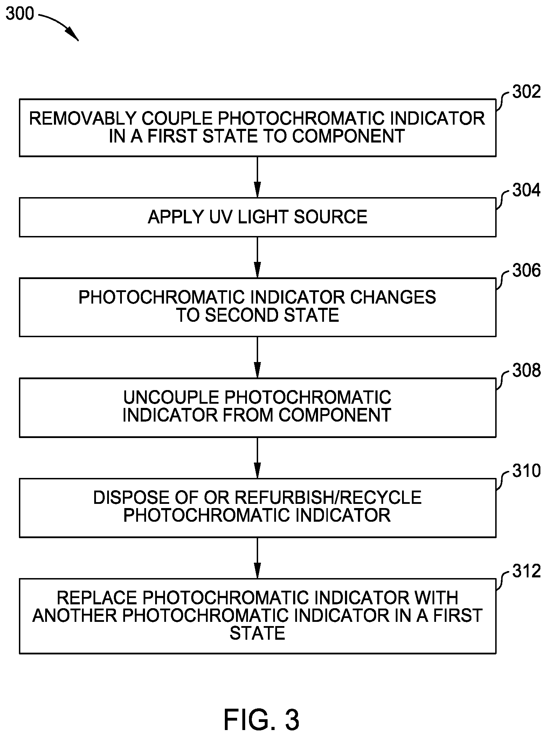

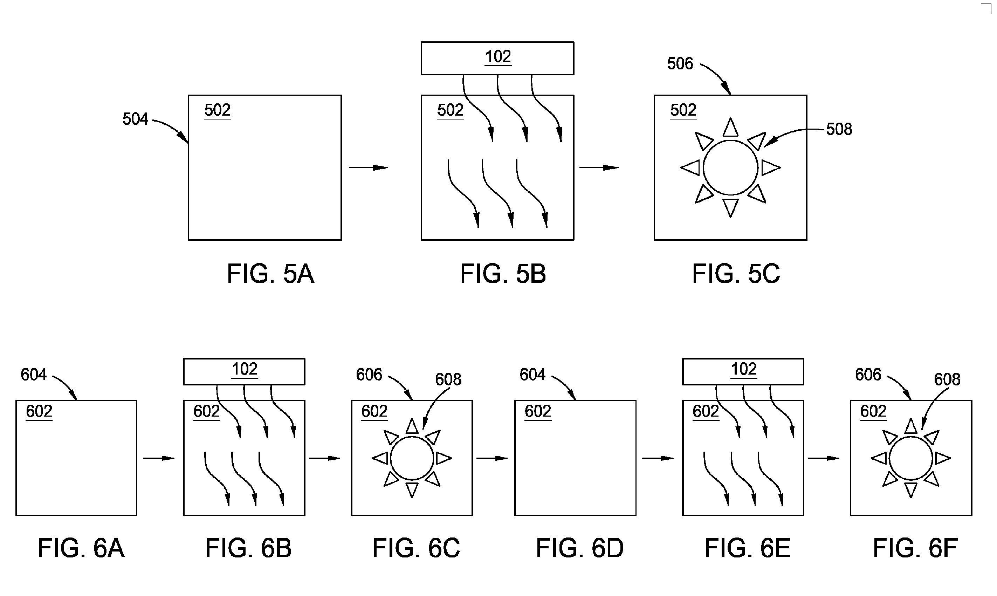

FIG. 3 depicts a flow chart of a method of cleaning 300 using photochromatic indicators according to examples of the present disclosure. FIGS. 5A-5C illustrate the photochromatic indicator at various operations in the method 300. At operation 302, a photochromatic indicator in a first state is removably coupled to a component to be cleaned (302- Removably couple photochromatic indicator in a first state to component). FIG. 5A illustrates one example the first state 504 of the photochromatic indicator 502 where no brand identifier or other marks are visible. The removable coupling at operation 302 can occur using an adhesive, a mechanical means, a magnetic means, a hook-and-loop means, a friction or press-fit-based means, or a combination of means. At operation 304 a UV light source is applied to the component including the photochromatic indicator (304 - Apply UV light source). In specific examples, UV light sources such as wands having, for example wavelengths of from about 275 to about 395 nm can be used. In other examples, UV light sources such as wands having wavelengths from about 260 to about 285 nm can be used. In still other examples, UV light sources such as wands having wavelengths from about 222 nm to about 254 nm can be used.

The UV light source may be applied to the component including the photochromatic indicator for a predetermined period of time and at a predetermined wavelength. In one aspect, the predetermined period of time can be from about 5 seconds to about 2 minutes. In another aspect, the predetermined period of time can be from about 10 seconds to about 60 seconds. In yet another aspect, the predetermined period of time can be from about 15 seconds to about 45 seconds. The UV wavelength used at operation 304 can be from about 100 nm to about 400 nm. In one example, UVA is used, having a wavelength from about 315 nm to about 400 nm. In another example, UVB is used, having a wavelength from about 280 nm to about 314 nm. In yet another example, UVC is used, having a wavelength from about 10 0nm to about 279 nm. In still another example, the UV wavelength used at operation 304 is from about 222 nm to about 254 nm. In other examples, combinations or overlapping ranges of UVA, UVB, and UVC may be used.

FIG. 5B illustrates the application of a UV light source (102) to the photochromatic indicator 502. At operation 306, in response to the photochromatic indicator absorbing the UV wavelengths from the UV light source 102, the photochromatic indicator changes from the first state (504) to the second state (506 as shown in FIG. 5C) (306-Photochromatic indicator changes to second state). The change from the first state to the second state can be confirmed visually by the naked eye, e.g., without magnification, black light, or other visual aid or visual assistance. In the example in FIG. 5C, the application of the UV light source 102 causes the graphic 508 to appear in photochromatic pigment when the photochromatic indicator 502 is in the second state 506.

In other examples, mobile devices including apps configured to confirm the presence of a graphic or a color change, or both, may be used to confirm the change from the first state to the second state. In still other examples, a tactile sensation may be used to confirm the change from the first state to the second state. In this example, the photochromatic indicator includes irreversible pigment and is intended for single-use. The photochromatic indicator may take the form of a seat cover, armrest cover, headrest cover, tray table cover, power outlet cover, touch-screens or touch screen covers or cases, table cover, retail or restaurant counter cover, or other disposable cover used for travel, retail, food service, financial institution, or in a shared workspace such as a conference room or other workspace that may be used by different individuals. The second state indicates that the component has been cleaned and can be used, for example, for a flight, a train ride, a bus ride, or a gaming/e-sports experience. Accordingly, subsequent to the use of the associated component, at operation 308, the photochromatic indicator is uncoupled from the component (308 - Uncouple photochromatic indicator from component) and disposed of at operation 310 (310 - Dispose of or refurbish/recycle photochromatic indicator). In some examples, instead of being disposed of at operation 310, the photochromatic indicator may be refurbished or recycled, depending upon its constituent material(s). At operation 312, a new, refurbished, or recycled photochromatic indicator in the first state is removably coupled to the component (312- Replace photochromatic indicator with another photochromatic indicator in a first state).

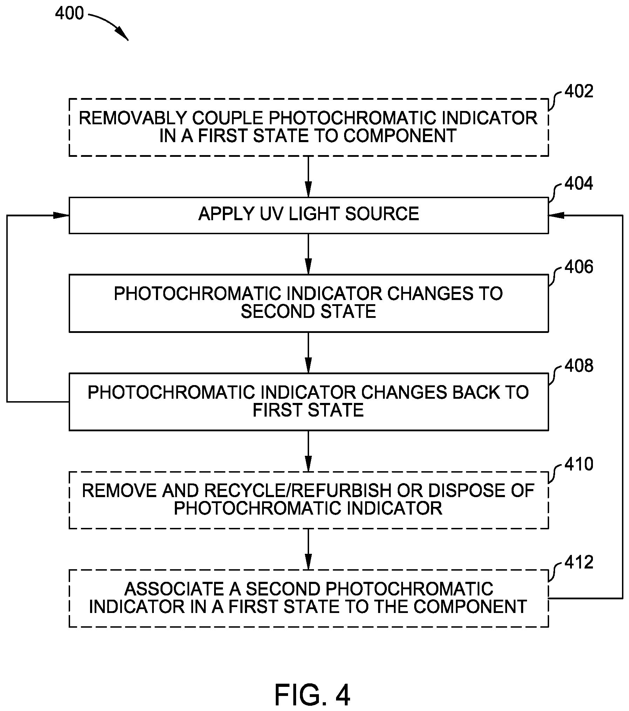

FIG. 4 is an illustration of a flow chart of a method of cleaning 400 using photochromatic indicators. FIGS. 6A-6F illustrate the photochromatic indicator at various operations of the method 400. In the method 400, at optional operation 402, a photochromatic indicator in a first state is removably coupled to a component to be cleaned (402 - Removably couple photochromatic indicator in a first state to component). The removable coupling at operation 402 can occur using an adhesive, a mechanical means, a magnetic means, a hook-and-loop means, a friction or press-fit-based means, or a combination of means. Operation 402 is optional because, in some examples, the photochromatic indicator is formed integrally with the component or is a semi-permanent or permanent aspect of the component. FIG. 6A illustrates one example the first state 604 of the photochromatic indicator 602 where no brand identifier or other marks are visible to the naked eye.

At operation 404, a UV light source is applied to the component including the photochromatic indicator (304 - Apply UV light source). The UV light source may be applied to the component including the photochromatic indicator for a predetermined period of time and at a predetermined wavelength. In one aspect, the predetermined period of time can be from about 5 seconds to about 2 minutes. In another aspect, the predetermined period of time can be from about 10 seconds to about 60 seconds.

In yet another aspect, the predetermined period of time can be from about 15 seconds to about 45 seconds. The UV wavelength used at operation 404 can be from about 100 nanometers (nm) to about 400 nm. In one example, UVA is used, having a wavelength from about 315 nm to about 400 nm. In another example, UVB is used, having a wavelength from about 280 nm to about 314 nm. In yet another example, UVC is used, having a wavelength from about 100 nm to about 279 nm. In other examples, overlapping ranges of UVA, UVB, and UVC wavelengths may be used at operation 404. In specific examples, UV light sources such as wands having, for example wavelengths of from about 275 to about 395 nm can be used. In other examples, UV light sources such as wands having wavelengths from about 260 to about 285 nm can be used. In still other examples, UV light sources such as wands having wavelengths from about 222 nm to about 254 nm can be used.

FIG. 6B illustrates the application of the UV light source to the photochromatic indicator 602. At operation 406, in response to the photochromatic indicator absorbing the UV wavelengths from the UV light source 102, the photochromatic indicator changes from the first state (604) to the second state (606 as shown in FIG. 6C) (406 - Photochromatic indicator changes to second state). The change from the first state to the second state can be confirmed visually by the naked eye, e.g., without magnification. In the example in FIG. 6C, the application of the UV light source 102 causes the graphic 608 to appear in photochromatic pigment when the photochromatic indicator 602 is in the second state 606. The photochromatic indicator 602 includes reversible photochromatic pigment. Accordingly, at operation 408, the photochromatic indicator 602 changes back to the first state 604 as shown in FIG. 6D (408 - Photochromatic indicator changes back to first state). This change at operation 408 can occur after a predetermined amount of time. Further, as discussed above, the photochromatic indicator 602 can include one or more graphics of a single pigment or of an pigment gradient that may indicate that the photochromatic indicator 602 is changing back to the first state 604 (FIG. 6D).

In this example, as indicated by the arrow from operation 408 to operation 404, the UV light source (such as the UV light source 102 as shown in FIGS. 6B and 6E) can be reapplied as shown in FIG. 6E to change the photochromatic indicator 602 back to the second state (606 in FIG. 6F) from the first state 604, such that the graphic 608 reappears in response to the application of the UV light source in each cycle indicated by the arrow. This cycle of applying the UV light source to the photochromatic indicator 602 to change it from the first state (604) to the second state (606) can occur in a plurality of iterations. The plurality of iterations may be a predetermined number from two to over 100, depending upon factors including the type of material from which the photochromatic indicator 602 is formed, the photochromatic pigment(s) used, the component the photochromatic indicator 602 is associated with, or other factors or combinations of factors.

In some examples of the method 400, at operation 410, at least one of the photochromatic indicator 602 or the component it is associated with can be removed and disposed of or refurbished (410 - Remove and recycle/refurbish or dispose of photochromatic indicator). Subsequently, at operation 412, another photochromatic indicator can be associated with the component, or with the replaced or refurbished component (412 - Associate a second photochromatic indicator in a first state to the component), and the method 400 can return to operation 404 such that a new plurality of cleaning iterations can be performed.

In various aspects of the present disclosure, the methods 300 and 400 can be executed by employees such as airline employees, and the cleaning results verified by the employees as well as the passengers. In other aspects, the methods 300 and 400 can be executed and visually confirmed by passengers or customers. In still other aspects of the present disclosure, the methods 300 and 400 can be executed automatically in response to commands transmitted to the system 100 on a predetermined schedule or in response to a triggering event such as deplaning or a shift change. In some aspects of the present disclosure, more than one graphic may be included in a photochromatic indicator, such that a first graphic changes from a first state to a second state in response to a first combination of a time and a UV wavelength, and a second graphic changes from a first state to a second state in response to a second combination of a time and a UV wavelength.

In the current disclosure, reference is made to various aspects. However, it should be understood that the present disclosure is not limited to specific described aspects. Instead, any combination of the above features and elements, whether related to different aspects or not, is contemplated to implement and practice the teachings provided herein. Additionally, when elements of the aspects are described in the form of "at least one of A and B," it will be understood that aspects including element A exclusively, including element B exclusively, and including element A and B are each contemplated. Furthermore, although some aspects may achieve advantages over other possible solutions and/or over the prior art, whether or not a particular advantage is achieved by a given aspect is not limiting of the present disclosure. Thus, the aspects, features, aspects and advantages disclosed herein are merely illustrative and are not considered elements or limitations of the appended claims except where explicitly recited in a claim(s). Likewise, reference to "the invention" shall not be construed as a generalization of any inventive subject matter disclosed herein and shall not be considered to be an element or limitation of the appended claims except where explicitly recited in a claim(s).

As will be appreciated by one skilled in the art, aspects described herein may be embodied as a system, method or computer program product. Accordingly, aspects may take the form of an entirely hardware aspect, an entirely software aspect (including firmware, resident software, micro-code, etc.) or an aspect combining software and hardware aspects that may all generally be referred to herein as a "circuit," "module" or "system." Furthermore, aspects described herein may take the form of a computer program product embodied in one or more computer readable storage medium(s) having computer readable program code embodied thereon.

Program code embodied on a computer readable storage medium may be transmitted using any appropriate medium, including but not limited to wireless, wireline, optical fiber cable, RF, etc., or any suitable combination of the foregoing.

Computer program code for carrying out operations for aspects of the present disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present disclosure are described herein with reference to flowchart illustrations and/or block diagrams of methods, apparatuses (systems), and computer program products according to aspects of the present disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the block(s) of the flowchart illustrations and/or block diagrams.

These computer program instructions may also be stored in a computer readable medium that can direct a computer, other programmable data processing apparatus, or other device to function in a particular manner, such that the instructions stored in the computer readable medium produce an article of manufacture including instructions which implement the function/act specified in the block(s) of the flowchart illustrations and/or block diagrams.

The computer program instructions may also be loaded onto a computer, other programmable data processing apparatus, or other device to cause a series of operational steps to be performed on the computer, other programmable apparatus or other device to produce a computer implemented process such that the instructions which execute on the computer, other programmable data processing apparatus, or other device provide processes for implementing the functions/acts specified in the block(s) of the flowchart illustrations and/or block diagrams.

The flowchart illustrations and block diagrams in the Figures illustrate the architecture, functionality, and operation of possible implementations of systems, methods, and computer program products according to various aspects of the present disclosure. In this regard, each block in the flowchart illustrations or block diagrams may represent a module, segment, or portion of code, which comprises one or more executable instructions for implementing the specified logical function(s). It should also be noted that, in some alternative implementations, the functions noted in the block may occur out of the order noted in the Figures. For example, two blocks shown in succession may, in fact, be executed substantially concurrently, or the blocks may sometimes be executed in the reverse order or out of order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

Also provided are the following illustrative, non-exhaustive examples of further non-claimed embodiments that are compatible with the claimed subject matter:
In the method according to claim 14, the photochromatic indicator may be included in a single-use component removably coupled to the component through an adhesive, a magnetic means, a hook-and-loop means, a mechanical means, or combinations thereof.

In the claimed method, the photochromatic indicator may include two or more photochromatic pigments, each of the two or more photochromatic pigments having an at least one of a different concentration or a different composition.

The method according to claim 15 may further comprise, prior to applying the UV light source, removably coupling the first photochromatic indicator to the component (402), the first photochromatic indicator including irreversible photochromatic pigment.

The claimed method may further comprise performing (a) and (b) for a plurality of iterations, wherein the first photochromatic indicator includes at least one reversible pigment.

The claimed method may further comprise, subsequent to performing (a) and (b) for a plurality of iterations:
uncoupling the first photochromatic indicator from the component (410);
disposing of the first photochromatic indicator (410); and
associating a second photochromatic indicator with the component, the second photochromatic indicator being in the first state (412).

In the claimed method, the first photochromatic indicator may comprise a brand identifier.

In the claimed assembly or system, the photochromatic indicator (110A) may be configured as a disposable, single-use element.

In the claimed assembly or system, the photochromatic indicator (110A) may be associated with the component (110) via a means selected from the group consisting of: an adhesive means, a mechanical means, a magnetic means, a hook-and-loop means, and combinations thereof.

While the foregoing is directed to aspects of the present disclosure, other and further aspects of the disclosure may be devised without departing from the basic scope thereof, and the scope thereof is determined by the claims that follow.

## Claims

1. An assembly comprising:
a photochromatic indicator (110A) associated with a component (110), the photochromatic indicator (110A) being in a first state under ambient light and configured to be in a second state under the ambient light after being exposed to a UV light source (102), wherein the first state is different than the second state, the photochromic indicator (110A) being configured to change from the first state to the second state in response to the UV light source (102) to indicate that the component (110) has been cleaned, **characterised in that**
the photochromatic indicator (110A) comprises a plurality of apertures (204, 206, 208, 210, 212, 214) of various geometries configured to allow access to a control or selection feature of the respective component (110) associated with the photochromatic indicator (110A).

2. The assembly according to claim 1, wherein the various geometries of the plurality of apertures (204, 206, 208, 210, 212, 214) include a circular aperture (204), a polygonal aperture (208), a triangular aperture (210), a horizontal slot aperture (212), or a vertical slot aperture (214).

3. The assembly of claim 1 or 2, wherein the component (110) is formed from a material selected from the group consisting of: a polymer, a polycarbonate, a metal, a composite, a nano-reinforced material, an organic material, a textile fabric, and combinations thereof.

4. The assembly of any of claims 1 to 3, wherein the photochromatic indicator (110A) is in a form selected from the group consisting of: a sticker, a thread, a textile fabric, an organic material, a polycarbonate element, a polymer element, a metal element, a coating, and combinations thereof.

5. The assembly of any of claims 1 to 4, wherein the photochromatic indicator (110A) is included in a tray cover, a headrest cover, a seat cover, an armrest cover, an outlet cover, or a headset cover.

6. The assembly of any of claims 1 to 5, wherein the photochromatic indicator (110A) includes a reversible or irreversible photochromatic pigment.

7. The assembly of any of claims 1 to 6, wherein the photochromatic indicator (110A) is not visible to the naked eye when in the first state.

8. The assembly of any of claims 1 to 7, wherein the photochromatic indicator (110A) is included in a cover comprising at least one aperture, wherein the cover is configured to couple to the component (110) through one or more of an adhesive, a magnetic means, a hook-and-loop means, a mechanical means, or combinations thereof.

9. The assembly of claim 8, wherein the component (110) comprises a gaming system.

10. A system, comprising:
an ultraviolet (UV) light source (102);
a power source (104) configured to provide power to the UV light source (102); and
an assembly comprising the photochromatic indicator (110A) associated with the component (110) according to claim 1.

11. The system of claim 10, wherein the UV light source (102) includes a wireless device, a wired device, a device removably coupled to a wall of an aircraft passenger cabin, a device removably coupled to a ceiling of an aircraft passenger cabin, a device removably coupled to a seat of an aircraft passenger cabin, or a device removably coupled to a floor of an aircraft passenger cabin.

12. The system of claim 10 or 11, wherein the UV light source (102) is configured to emit a wavelength from about 222 nanometers (nm) to about 254 nm.

13. A method of cleaning, comprising:
removably coupling a first photochromatic indicator to a component (302), the first photochromatic indicator being in a first state;
applying an ultraviolet (UV) light source to the component (304), wherein, in response to the UV light source, the first photochromatic indicator changes from the first state to a second state (306), the second state indicating that the component has been cleaned, wherein said second state is visible to a naked eye under ambient light;
uncoupling the first photochromatic indicator from the component (308);
disposing of the first photochromatic indicator (310); and
removably coupling a second photochromatic indicator to the component (312), the photochromatic indicator being in the first state, wherein each of the first and second photochromatic indicator comprises a plurality of apertures (204, 206, 208, 210, 212, 214) of various geometries configured to allow access to a control or selection feature of the respective component onto which the photochromatic indicator is removably coupled.

14. A method of cleaning, comprising:
(a) applying an ultraviolet (UV) light source to a component associated with a first photochromatic indicator in a first state (404),
wherein, in response to the UV light source being applied to the first photochromatic indicator and the first photochromatic indicator absorbing energy from the UV light source, the first photochromatic indicator changes from the first state to a second state (406), the second state indicating that the component has been cleaned; and
after a predetermined period of time, in response to the energy from the UV light source absorbed by the first photochromatic indicator dissipating, the first photochromatic indicator changing from the second state to the first state (408), wherein the first photochromatic indicator comprises a plurality of apertures (204, 206, 208, 210, 212, 214) of various geometries configured to allow access to a control or selection feature of the respective component associated with the first photochromatic indicator.

15. The method of claim 14 further comprising:
(b) re-applying the UV light source to the first photochromatic indicator subsequent to the first photochromatic indicator changing from the second state to the first state (404), wherein the first photochromatic indicator changes from the first state back to the second state in response to (b) (406), indicating that the component has been cleaned.

## Patentansprüche

1. Anordnung aufweisend:
einen fotochromatischen Indikator (110A), der mit einer Komponente (110) assoziiert ist, wobei der fotochromatische Indikator (110A) unter Umgebungslicht in einem ersten Zustand ist und konfiguriert ist, unter dem Umgebungslicht in einem zweiten Zustand zu sein, nachdem er einer UV-Lichtquelle (102) ausgesetzt worden ist, wobei der erste Zustand sich von dem zweiten Zustand unterscheidet, wobei der fotochromatische Indikator (110A) konfiguriert ist, als Antwort auf die UV-Lichtquelle (102) aus dem ersten Zustand in den zweiten Zustand zu wechseln, um anzuzeigen, dass die Komponente (110) gereinigt worden ist, **dadurch gekennzeichnet, dass**
der fotochromatische Indikator (110A) mehrere Öffnungen (204, 206, 208, 210, 212, 214) verschiedenartiger Geometrien aufweist, die konfiguriert sind, Zugang zu einem Steuer- oder Auswahlmerkmal der mit dem fotochromatischen Indikator (110A) assoziierten jeweiligen Komponente (110) zu erlauben.

2. Anordnung nach Anspruch 1, wobei die verschiedenartigen Geometrien der mehreren Öffnungen (204, 206, 208, 210, 212, 214) eine kreisförmige Öffnung (204), eine polygonale Öffnung (208), eine dreieckige Öffnung (210), eine horizontale Schlitzöffnung (212), oder eine vertikale Schlitzöffnung (214) aufweisen.

3. Anordnung nach Anspruch 1 oder 2, wobei die Komponente (110) aus einem aus der folgenden Gruppe ausgewählten Material besteht: ein Polymer, ein Polycarbonat, ein Metall, ein Verbundwerkstoff, ein nano-verstärktes Material, ein organisches Material, ein Textilstoff, und Kombinationen davon.

4. Anordnung nach einem der Ansprüche 1 bis 3, wobei der fotochromatische Indikator (110A) in einer aus der folgenden Gruppe ausgewählten Ausführung vorliegt: ein Aufkleber, ein Faden, ein Textilstoff, ein organisches Material, ein Polycarbonat-Element, ein Polymer-Element, ein Metallelement, eine Beschichtung, und Kombinationen davon.

5. Anordnung nach einem der Ansprüche 1 bis 4, wobei der fotochromatische Indikator (110A) in einer Ablageabdeckung, einem Kopfstütze-Überzug, einem Sitzüberzug, einem Armlehne-Überzug, einer Verkaufshülle, oder einer Headset-Hülle enthalten ist.

6. Anordnung nach einem der Ansprüche 1 bis 5, wobei der fotochromatische Indikator (110A) ein reversibles oder irreversibles fotochromatisches Pigment aufweist.

7. Anordnung nach einem der Ansprüche 1 bis 6, wobei der fotochromatische Indikator (110A), wenn in dem ersten Zustand, mit dem bloßen Auge nicht erkennbar ist.

8. Anordnung nach einem der Ansprüche 1 bis 7, wobei der fotochromatische Indikator (110A) in einer Hülle enthalten ist, die mindestens eine Öffnung aufweist, wobei die Hülle konfiguriert ist, mithilfe eines Klebstoffs, einer magnetischen Einrichtung, einer Klett-Einrichtung, einer mechanischen Einrichtung, oder Kombinationen davon an die Komponente (110) gekoppelt zu werden.

9. Anordnung nach Anspruch 8, wobei die Komponente (110) ein Spielsystem aufweist.

10. System, aufweisend:
eine Ultraviolett (UV) Lichtquelle (102);
eine Energiequelle (104), die konfiguriert ist, der UV-Lichtquelle (102) Energie bereitzustellen; und
eine Anordnung aufweisend den mit der Komponente (110) assoziierten fotochromati-schen Indikator (110A) nach Anspruch 1.

11. System nach Anspruch 10, wobei die UV-Lichtquelle (102) eine Drahtlosvorrich-tung, eine verdrahte Vorrichtung, eine lösbar an eine Wand einer Flugzeug-Passagierkabine gekoppelte Vorrichtung, eine lösbar an eine Decke einer Flugzeug-Passagierkabine gekoppelte Vorrichtung, eine lösbar an einen Sitz einer Flugzeug-Passagierkabine gekoppelte Vorrichtung, oder eine lösbar an einen Boden einer Flugzeug-Passagierkabine gekoppelte Vorrichtung auf-weist.

12. System nach Anspruch 10 oder 11, wobei die UV-Lichtquelle (102) konfiguriert ist, eine Wellenlänge von ungefähr 222 Nanometer (nm) bis ungefähr 254nm zu emittieren.

13. Reinigungsverfahren, aufweisend:
lösbares Koppeln eines ersten fotochromatischen Indikators an eine Komponente (302), wobei der fotochromatische Indikator in einem ersten Zustand ist;
Anwenden einer Ultraviolett (UV) Lichtquelle auf die Komponente (304), wobei als Antwort auf die UV-Lichtquelle der erste fotochromatische Indikator aus dem ersten Zustand in einen zweiten Zustand (306) wechselt, wobei der zweite Zustand anzeigt, dass die Kompo-nente gereinigt worden ist, wobei der zweite Zustand unter Umgebungslicht mit dem bloßen Auge erkennbar ist;
Abkoppeln des ersten fotochromatischen Indikators von der Komponente (308);
Entsorgen des ersten fotochromatischen Indikators (310); und
lösbares Koppeln eines zweiten fotochromatischen Indikators an die Komponente (312), wobei der fotochromatische Indikator in dem ersten Zustand ist, wobei sowohl der erste als auch der zweite fotochromatische Indikator mehrere Öffnungen (204, 206, 208, 210, 212, 214) verschiedenartiger Geometrien aufweist, die konfiguriert sind, Zugang zu einem Steuer- oder Auswahlmerkmal der jeweiligen Komponente zu erlauben, an die der fotochromatische Indikator lösbar gekoppelt ist.

14. Reinigungsverfahren, aufweisend:
(a) Anwenden einer Ultraviolett (UV) Lichtquelle auf eine Komponente, die mit einem ersten fotochromatischen Indikator in einem ersten Zustand assoziiert ist (404),
wobei als Antwort darauf, dass die UV-Lichtquelle auf den ersten fotochromatischen Indikator angewendet worden ist und der erste fotochromatische Indikator Energie aus der UV-Lichtquelle absorbiert, der erste fotochromatische Indikator aus dem ersten Zustand in einen zweiten Zustand wechselt (406), wobei der zweite Zustand anzeigt, dass die Komponente gereinigt worden ist; und
als Antwort auf Dissipation der von dem ersten fotochromatischen Indikator aus der UV-Lichtquelle absorbierten Energie der erste fotochromatische Indikator nach einer vorgegebenen Zeitdauer aus dem zweiten Zustand in den ersten Zustand wechselt (408), wobei der erste fotochromatische Indikator mehrere Öffnungen (204, 206, 208, 210, 212, 214) verschie-denartiger Geometrien aufweist, die konfiguriert sind, Zugang zu einem Steuer- oder Aus-wahlmerkmal der mit dem ersten fotochromatischen Indikator assoziierten jeweiligen Kompo-nente zu erlauben.

15. Verfahren nach Anspruch 14, ferner aufweisend:
(b) erneutes Anwenden der UV-Lichtquelle auf den ersten fotochromatischen Indikator nach dem Wechsel des ersten fotochromatischen Indikators aus dem zweiten Zustand in den ersten Zustand (404), wobei als Antwort auf (b) der erste fotochromatische Indikator aus dem ersten Zustand zurück in den zweiten Zustand wechselt (406), was anzeigt, dass die Kompo-nente gereinigt worden ist.

## Revendications

1. Ensemble comprenant :
un indicateur photochromatique (110A) associé à un composant (110), l'indicateur photochromatique (110A) étant dans un premier état sous une lumière ambiante et configuré pour être dans un deuxième état sous la lumière ambiante après avoir été exposé à une source de lumière UV (102), dans lequel le premier état est différent du deuxième état, l'indicateur photochromatique (110A) étant configuré pour passer du premier état au deuxième état en réponse à la source de lumière UV (102) pour indiquer que le composant (110) a été nettoyé, **caractérisé en ce que**
l'indicateur photochromatique (110A) comprend une pluralité d'ouvertures (204, 206, 208, 210, 212, 214) de différentes géométries configurées pour permettre l'accès à une caractéristique de commande ou de sélection du composant (110) respectif associé à l'indicateur photochromatique (110A).

2. Ensemble selon la revendication 1, dans lequel les différentes géométries de la pluralité d'ouvertures (204, 206, 208, 210, 212, 214) comportent une ouverture circulaire (204), une ouverture polygonale (208), une ouverture triangulaire (210), une ouverture en fente horizontale (212) ou une ouverture en fente verticale (214).

3. Ensemble selon la revendication 1 ou 2, dans lequel le composant (110) est formé d'un matériau choisi dans le groupe consistant en : un polymère, un polycarbonate, un métal, un composite, un matériau nano-renforcé, un matériau organique, un tissu textile et leurs combinaisons.

4. Ensemble selon l'une quelconque des revendications 1 à 3, dans lequel l'indicateur photochromatique (110A) est sous une forme choisie dans le groupe consistant en : un autocollant, un fil, un tissu textile, un matériau organique, un élément polycarbonate, un élément polymère, un élément métallique, un revêtement et leurs combinaisons.

5. Ensemble selon l'une quelconque des revendications 1 à 4, dans lequel l'indicateur photochromatique (110A) est inclus dans un couvre-plateau, un protège-appuie-tête, une housse de siège, un protège-accoudoir, un cache-prise ou une housse de casque.

6. Ensemble selon l'une quelconque des revendications 1 à 5, dans lequel l'indicateur photochromatique (110A) comporte un pigment photochromatique réversible ou irréversible.

7. Ensemble selon l'une quelconque des revendications 1 à 6, dans lequel l'indicateur photochromatique (110A) n'est pas visible à l'oeil nu lorsqu'il est dans le premier état.

8. Ensemble selon l'une quelconque des revendications 1 à 7, dans lequel l'indicateur photochromatique (110A) est inclus dans un revêtement comprenant au moins une ouverture, dans lequel le revêtement est configuré pour être couplé au composant (110) par l'intermédiaire d'un ou plusieurs parmi un adhésif, un moyen magnétique, un moyen de fermeture autoagrippante, un moyen mécanique ou leurs combinaisons.

9. Ensemble selon la revendication 8, dans lequel le composant (110) comprend un système de jeu.

10. Système, comprenant :
une source de lumière ultraviolette (UV) (102) ;
une source d'alimentation (104) configurée pour fournir de l'énergie à la source de lumière UV (102) ; et
un ensemble comprenant l'indicateur photochromatique (110A) associé au composant (110) selon la revendication 1.

11. Système selon la revendication 10, dans lequel la source de lumière UV (102) comporte un dispositif sans fil, un dispositif câblé, un dispositif couplé de manière amovible à une paroi d'une cabine passagers d'aéronef, un dispositif couplé de manière amovible à un plafond d'une cabine passagers d'aéronef, un dispositif couplé de manière amovible à un siège d'une cabine passagers d'aéronef ou un dispositif couplé de manière amovible à un plancher d'une cabine passagers d'aéronef.

12. Système selon la revendication 10 ou 11, dans lequel la source de lumière UV (102) est configurée pour émettre une longueur d'onde d'environ 222 nanomètres (nm) à environ 254 nm.

13. Procédé de nettoyage, comprenant :
le couplage amovible d'un premier indicateur photochromatique à un composant (302), le premier indicateur photochromatique étant dans un premier état ;
l'application d'une source de lumière ultraviolette (UV) sur le composant (304), dans lequel, en réponse à la source de lumière UV, le premier indicateur photochromatique passe du premier état à un deuxième état (306), le deuxième état indiquant que le composant a été nettoyé, dans lequel ledit deuxième état est visible à l'oeil nu sous une lumière ambiante ;
le découplage du premier indicateur photochromatique du composant (308) ;
l'élimination du premier indicateur photochromatique (310) ; et
le couplage amovible d'un deuxième indicateur photochromatique au composant (312), l'indicateur photochromatique étant dans le premier état, dans lequel chacun du premier et du deuxième indicateur photochromatique comprend une pluralité d'ouvertures (204, 206, 208, 210, 212, 214) de différentes géométries configurées pour permettre l'accès à une caractéristique de commande ou de sélection du composant respectif sur lequel l'indicateur photochromatique est couplé de manière amovible.

14. Procédé de nettoyage, comprenant :
(a) l'application d'une source de lumière ultraviolette (UV) sur un composant associé à un premier indicateur photochromatique dans un premier état (404),
dans lequel, en réponse à l'application de la source de lumière UV sur le premier indicateur photochromatique et à l'absorption par le premier indicateur photochromatique d'une énergie provenant de la source de lumière UV, le premier indicateur photochromatique passe du premier état à un deuxième état (406), le deuxième état indiquant que le composant a été nettoyé ; et
après une période de temps prédéterminée, en réponse à la dissipation de l'énergie provenant de la source de lumière UV absorbée par le premier indicateur photochromatique, le premier indicateur photochromatique passe du deuxième état au premier état (408), dans lequel le premier indicateur photochromatique comprend une pluralité d'ouvertures (204, 206, 208, 210, 212, 214) de différentes géométries configurées pour permettre l'accès à une caractéristique de commande ou de sélection du composant respectif associé au premier indicateur photochromatique.

15. Procédé selon la revendication 14 comprenant en outre :
(b) la réapplication de la source de lumière UV sur le premier indicateur photochromatique suite au passage du premier indicateur photochromatique du deuxième état au premier état (404), dans lequel le premier indicateur photochromatique repasse du premier état au deuxième état en réponse à (b) (406), indiquant que le composant a été nettoyé.
